# EUROPEAN PATENT APPLICATION

(11) **EP 3 351 094 A1**
(43) Date of publication of application: **25.07.2018**
(21) Application number: 17152528.0
(22) Date of filing: 20.01.2017
(51) Int. Cl.: A01K 67/027, C07K 14/535, C12N 9/22, C12N 15/85

(54) **ANIMAL MODEL FOR DRUG DEVELOPMENT**

(71) Applicant: Universitätsklinikum Hamburg-Eppendorf, 20246 Hamburg (DE)
(72) Inventor: Fuchs, Tobias, 20249 Hamburg (DE); Jiménez-Alcázar, Miguel, 20251 Hamburg (DE); Rangaswamy, Chandini, 20251 Hamburg (DE)
(74) Representative: Uexküll & Stolberg

(57) **Abstract**

The present invention relates to a non-human mammalian animal which has been modified to have in the blood, plasma and/or serum (a) an increased number of leukocytes and/or neutrophils, and (b) a reduced activity of the DNase 1 and/or DNase 1-like 3 enzymes. The non-human mammalian animal is particularly suitable for studying inflammation and/or a disease associated with inflammation. In a further aspect, the invention relates to the use of the non-human mammalian animal as a model for identifying therapeutic or diagnostic targets of inflammation and/or a disease associated with inflammation. In a still further aspect, the invention relates the use of the non-human mammalian animal as a model for drug candidate testing. In addition, a method for testing an anti-inflammatory drug candidate against extracellular DNA is provided. Finally, a method for testing an anti-inflammatory drug candidate for modifying the formation or degradation of neutrophil extracellular traps is provided.

## Description

The present invention relates to a non-human mammalian animal which has been modified to have in the blood, plasma and/or serum (a) an increased number of leukocytes and/or neutrophils, and (b) a reduced activity of the DNase 1 and/or DNase 1-like 3 enzymes. The non-human mammalian animal is particularly suitable for studying inflammation and/or a disease associated with inflammation. In a further aspect, the invention relates to the use of the non-human mammalian animal as a model for identifying or validating targets for diagnostic or therapeutic agents, preferably agents useful for diagnosing or treating inflammation and/or a disease associated with inflammation.

In a still further aspect, the invention relates the use of the non-human mammalian animal as a model for drug candidate testing. Finally, a method for testing an anti-inflammatory drug candidate for modifying the formation or degradation of neutrophil extracellular traps is provided.

### FIELD OF THE INVENTION

Inflammation is a host response to clear invaded microbes and restore injured tissue (Kolaczkowska, et al. (2013) Nat. Rev. Immunol. 13 (3): 159-75). Exacerbated and persistent inflammation damages host cells leading to morbidity and mortality in patients (Nathan, et al. (2010) Cell 140 (6): 871-82).

Polymorphonuclear neutrophils, short neutrophils or PMNs, are the most abundant white blood cells and the predominant cell type in inflammatory reactions (Kumar, et al. Robbins & Cotran Pathologic Basis of Disease, 2009). The life of a neutrophil begins in the bone marrow with the commitment of a hematopoietic stem cell to the myeloid linage. During the development of a myeloblast to a mature neutrophil the cytoplasm fills with storage granules, which carry ready-made enzymes and peptides. Further in neutrophil development, euchromatin shifts progressively to highly condensed and transcriptionally inactive heterochromatin, which is stored in a multi-lobulated nucleus in mature cells. The production and the mobilization of neutrophils are controlled by the granulocyte colony-stimulating factor (G-CSF). G-CSF stimulates the massive proliferation of granulocytic precursors and release of mature cells from the bone marrow into circulation.

Neutrophils entering the sinus of the bone marrow are short-lived and terminally differentiated cells. In blood, neutrophils are not capable of cell division or differentiation, but are fully equipped to promptly take part in the inflammatory response. Indeed, neutrophils are the first leukocytes, which are recruited to sites of inflammation. Along with macrophages, neutrophils are considered professional phagocytes. The phagocytic uptake is a well-documented, specialized mechanism, which allows the efficient destruction of internalized material.

In the last decade, neutrophil extracellular traps (NETs), extracellular DNA-filaments decorated with histones and granular proteins, were described to belong to the antimicrobial repertoire of neutrophils (Brinkmann, et al. (2004) Science 303 (5663): 1532-5.). However, uncontrolled or spontaneous NET-formation injures host cells and activates intravascular blood clotting under sterile conditions in patients with inflammatory diseases including autoimmune diseases, cardiovascular and thromboembolic diseases, neurodegenerative diseases, and cancer (Kolaczkowska, et al. (2013) Nat. Rev. Immunol. 13 (3): 159-75; Engelmann, et al. (2013) Nat. Rev. Immunol. 13 (1): 34-45). Host mechanisms, which disarm NETs to prevent tissue injury and thrombosis during inflammation, are not known at present.

Drugs, which modify the formation and/or degradation of NETs could enable therapies to prevent or ameliorate the adverse effects of inflammation. However, only one out of 5.000-10.000 drug candidates that enter the drug development pipeline will also enter the market (Hughes et al. (2011) British Journal of Pharmacology 162 (6): 1239-1249).

The vast majority of drug candidates fail in clinical trials, a late stage of drug development, causing substantial loss of investments. The lack of safety and efficacy accounts for approximately 90% of drug failures during clinical trials (Kola & Landis (2004) Nat Rev Drug Discov 3 (8):711-5). Therefore current preclinical animal models fail to predict the effectiveness of drug candidates in clinical development. Consequently, there is a need for animal models, which more closely reflect conditions of diseased patients. The present invention provides such a model and also methods for its use in inflammatory diseases.

### DESCRIPTION OF THE INVENTION

One reason for the inefficiency of current murine models of inflammatory disease may be the discrepancy in the number of circulating neutrophils in mice and humans. While humans contain on average 5 million neutrophils in one milliliter of blood, less than 0.5 million neutrophils are founds in one milliliter of blood of laboratory mice. Therefore, the animal model of the invention has been modified to increase the numbers of leukocytes and/or neutrophils in the blood of said animal.

To increase the numbers of leukocytes and/or neutrophils in the blood of the animal, copies of the gene *CSF3,* which encodes G-CSF, were introduced into mice and provided for the overexpression of said gene. Overexpression was accomplished by injecting a liver-specific expression plasmid containing the *CSF3* coding sequence in mice using the method of hydrodynamic tail vein injection. High levels of G-CSF were detectable in plasma 3 days after CSF3 administration and the concentration of circulating neutrophils increased steadily henceforward. After 2 weeks, 40-fold higher neutrophil counts were detected in G-CSF-overexpressing mice when compared to animals injected with the control plasmid lacking CSF3. Neutrophilic mice developed increased numbers of resident neutrophils in vital organs, splenomegaly, but grew normally, did not show signs of organ injury, and were macroscopically indistinguishable from untreated animals.

Another discrepancy between mice and humans has been identified in the course of the invention. The DNA-degrading activity of human plasma or serum is strongly reduced compared to the DNA-degrading activity of plasma or serum from laboratory mice. Consequently, the *in vitro* degradation of NETs by murine plasma or serum has been found to be strongly increased compared the degradation by human plasma or serum. In addition, it was found that extracellular DNase 1 (DNase1) or DNase 1-like 3 (DNase1l3) in the blood are sufficient to degrade NETs in vitro and in vivo. DNase 1 is present in serum at steady-state-conditions and expressed by non-hematopoietic tissues, including endocrine and exocrine glands (Napirei et al. (2004), The Biochemical journal, 380: 929-937). DNase1l3, also known as DNase-Gamma, is constitutively secreted into circulation by macrophages and dendritic cells (Sisirak et al. (2016), Cell, 166: 88-101; Mizuta, et al. (2013) PloS one 8 (12): e80223.).

In the course of the invention, mice lacking both DNases (DKO) were generated and DNA-degrading activity was detected in WT, DNase1^{-/-}, and DNase1l3^{-/-} sera, but not in DKO sera. Analysis of NET-degradation *in vitro* showed that NETs are stable in DKO-sera, whereas sera from WT, DNase1^{-/-}, and DNase1l3^{-/-} mice degraded NETs. Supplementation of DKO-serum with recombinant murine DNase1 or DNase1l3 restored the NET-degrading activity. *In vitro,* DNase1 preferentially cleaves protein-free DNA, while DNase1l3 targets DNA associated with proteins, such as polynucleosomes (Napirei, et al. (2005) Biochem. J. 389 (Pt 2): 355-64). To test whether DNase1 and DNase1l3 degrade NETs by distinct mechanisms, proteins in NETs were cross-linked using fixative. Fixed NETs were efficiently degraded by DNase1l3^{-/-} sera, but were resistant to degradation by DNase1^{-/-} sera. In line with these results, exposure of NETs to recombinant DNase1 or DNase1l3 medium showed that both DNases degrade naive NETs whereas fixed NETs are resistant to DNase1l3 activity. Collectively, these *in vitro* data identify extracellular DNase1 and DNase1l3 in murine serum as redundant NET-degrading enzymes.

The course of G-CSF-induced neutrophilia in WT mice was then compared to DNase1^{-/-}, DNase1l3^{-/-}, and DKO mice. Single KO mice were macroscopically indistinguishable from neutrophilic WT mice and untreated controls (not shown). All DKO mice died within 7 days after induction of G-CSF overexpression and developed a rapidly progressing and severe hypothermia with concomitant hematuria. No such phenotype was observed in DKO mice injected with the control plasmid. In order to conclude that the lack of both circulating DNases is responsible for this phenotype, several control experiments were performed. An off-target mutation has been recently reported for the DNase1^{-/-} mice used in this study (Rossaint, et al. (2014) Blood 123 (16): 2573-84). We therefore reproduced the phenotype in single and DKO mice derived from an independently generated DNase1^{-/-} strain (Bradley, et al. (2012) Mammalian genome 23 (9-10): 580-6.). DNase1- and DNase1l3-deficient mice spontaneously develop autoimmunity with features of lupus nephritis. Furthermore, DNase1 and/or DNase1l3 degrade nuclear material generated during cell death, and DNase1l3 has been implicated in B cell development (Shiokawa, et al. (2007) Cell Death Differ. 14 (5): 992-1000). To rule out actions of DNase1 and DNase1l3 other than degrading extracellular DNA, we treated DKO mice with a liver-specific expression plasmid containing the cDNA of DNase1 (pD1) or DNase1l3 (pD113). Both enzymes contain a secretion sequence, and consequently, hepatic expression of DNase1 or DNase1l3 restored their enzymatic activity in circulation as well as the NET-degrading activity of serum. Importantly, DKO mice expressing DNase1 or DNase1l3 in circulation survived G-CSF-induced neutrophilia, did not develop severe hypothermia or hematuria, and were macroscopically indistinguishable from untreated DKO mice.

Histological analysis of vital organs from hypothermic DKO mice revealed intravascular hematoxylin-rich clots, which fully or partially occluded blood vessels in lungs, liver, and kidneys. Untreated DKO mice, neutrophilic WT mice, and neutrophilic DKO mice expressing pD1 or pD1l3 did not show occluded blood vessels. Two distinct patterns in intravascular hematoxylin-rich bodies: dotted staining illustrating nuclei of leukocytes and diffuse staining pattern covering the space between nuclei. Positive staining with intercalating DNA dyes and antibodies against DNA-histone-complexes indicated extracellular chromatin as a major component of the clots. Extracellular chromatin was mainly derived from NETs, as evidenced by co-localization with markers of neutrophil granules such as cathelin-related antimicrobial peptide (CRAMP), myeloperoxidase, and citrullinated histones. Additionally, the DNA-clots contained von Willebrand factor (vWF) and fibrin , supporting the previously described pro-thrombotic activity of NETs. The formation of DNA-clots was associated with organ damage, as evidenced by increased levels of plasma lactate dehydrogenase (LDH). Elevated plasma levels of aminotransferease (ALT, AST), indicated liver damage, while high levels of blood urea nitrogen (BUN) and creatinine in plasma, and hematuria indicated renal failure. In addition, DKO mice developed anemia and thrombocytopenia. In summary, these data suggest that mice lacking DNase1 and DNase1l3 died of multi-organ damage induced by systemic intravascular DNA-clots comprising NETs.

NETs in murine and human tissue are predominantly identified as depositions of citrullinated histones (Brill, et al. (2012) J. Thromb. Haemost. 10 (1): 136-44; Savchenko, et al. (2014) J. Thromb. Haemost. 12 (6): 860-70.), a biomarker generated in neutrophils during NETosis, or as single extracellular DNA-filaments (von Bruhl, et al. (2012) J. Exp. Med. 209 (4): 819-35). Aggregates of extracellular DNA-filaments comprising NETs are formed in the synovial fluid of mice and patients with gout (Schauer, et al. (2014) Nat. Med. 20 (5): 511-7), but are unprecedented for other tissues. In the course of this invention sporadic clots of hematoxylin-positive filaments were identified in lung tissue from patients with pneumonia and sepsis (not shown). The clots were composed of NETs and resembled the appearance of DNA-clots observed in DKO mice with neutrophilia or endotoxemia.

The concentration of circulating DNase1 and DNase1l3 were compared in mice to humans. Murine plasma showed approximately 10-fold higher DNA-degrading activity than human plasma. Comparison of plasma from DNase1^{-/-} and DNase1l3^{-/-} mice to plasma from normal healthy donors (NHD) supplemented with α-human DNase1 antibodies and heparin, respectively, showed that activity of both DNases is approximately 10-fold higher in mice than in healthy donors.

In conclusion, these results identify DNases in circulation as host factors, which prevent vascular occlusion by NETs and thus maintain blood and tissue homeostasis during inflammation. The discrepancy in extracellular DNase activity between mice and humans likely adds to the inability of current murine models to predict the effectiveness of candidate drugs in humans reliably. While NETs are formed in humans and influence inflammation, they are rapidly degraded in the current murine models of inflammation. The present invention hence provides a murine model in which the activity of DNases in circulation is reduced.

Accordingly, in a first aspect the invention provides a non-human mammalian animal, which has been modified to have in the blood, plasma and/or serum
(a) an increased number of leukocytes and/or neutrophils, and
(b) a reduced activity of the DNase1 and/or DNase1l3 enzyme.

Preferably, the increase in the number of leukocytes and/or neutrophils and the reduction of activity of the DNase1 and/or DNase1l3 enzyme is such that NET formation can be observed.

The non-human mammalian animal can be any animal that is known to be useful for mimicking the inflammation of human tissue. The non-human mammalian animal can be a pig, cow, dog, cat, horse, donkey, goat, sheep, llama, or non-human primate (e.g. chimp). Preferably, the non-human mammalian animal is a rodent, such as a rat, mouse, hamster, rabbit, or guinea pig. In a particularly preferred aspect, the non-human mammalian animal is a mouse. For example, the mouse can have a C57BL/6 genetic background. Such mice can be purchased from different suppliers, e.g. from Charles River Laboratories.

The non-human mammalian animal has been modified to have a reduced activity of the enzymes DNase1 and/or DNase1l3 in the blood, plasma and/or serum. In a preferred embodiment, the activity of the enzymes of the animal is reduced by inactivation of the genes encoding these enzymes, by generating a knockout mouse. Methods for the generation of a knockout mouse are widely known in the art (Hall et al. (2009) Curr Protoc Cell Biol; Chapter 19: Unit 19.12 19.12.1-17). These methods usually include the construction of a targeting vector that harbors the modified sequence that will be introduced into murine embryonic stem cells. The knocked-out embryonic stem cells are then inserted into a mouse. The blastocyst is implanted into the uterus of female mice which ultimately results in chimeric offspring. Heterozygous mice are then interbred to provide mice that are homozygous for the knocked-out gene, i.e. they do not comprise any functional copy of the unmodified gene. Alternative methods for gene inactivation comprise the CRISPR/Cas9 system (Beil-Wagner et al. (2016) Sci Rep., 6: 21377).

Alternative methods may include the use of mice with spontaneous genetic mutations or acquired deficiencies of DNase1 and/or DNase1l3, e.g. strain MRL-lpr and strain NZB/w F1 hybrids (Seredkina et al. (2009), American Journal of Pathology, 175:97-106; Wilber et al. (2003), Clinical experimental Immunology, 134:46-52). Furthermore, mice may be treated with known inhibitors of DNase1 and/or DNase1l3 such as actin or heparin. In another embodiment, the activity of the DNase1 and DNase1l3 enzymes may be inhibited or even completely blocked by the administration of antibodies against the enzymes.

The non-human mammalian animal has been further modified to have an increased number of leukocytes and/or neutrophils in the blood, plasma and/or serum. The increase in the number of leukocytes and/or neutrophils is permanent which means that it lasts for at least 24 h, preferably at least 36 h, at least 48 h, at least 60 h, or at least 72 h, or longer. The increase in the number of leukocytes and/or neutrophils is at least 2-fold, preferably at least 5-fold, at least 6-fold, at least 7-fold, at least 8-fold, at least 9-fold, at least 10-fold, at least 15-fold, at least 20-fold, at least 25-fold, or more compared to an animal which has not been modified to increase the number of leukocytes and/or neutrophils, e.g. a wild-type animal of the same species and the same genetic background.

The increase number of leukocytes and/or neutrophils is preferably established by overexpressing an endogenous or exogenous nucleotide sequence that encodes a protein that is effective in increasing the number of leukocytes and/or neutrophils in the blood, plasma and/or serum of said animal. The sequence encoding the protein that is effective in increasing leukocytes and/or neutrophils can be a genomic sequence located in the genome of the non-human mammalian animal. Preferably, the sequence encoding the protein that is effective in mobilizing leukocytes and/or neutrophils is the granulocyte-stimulating factor (G-CSF), more preferably murine G-CSF.

In a preferred embodiment, the non-human mammalian animal has been modified to express the sequence encoding the protein that is effective in increasing leukocytes and/or neutrophils in the blood, plasma and/or serum permanently which means that the expression lasts for at least 24 h, preferably at least 36 h, at least 48 h, at least 60 h, or at least 72 h, or longer.

Methods for the overexpression of exogenous genes are widely known and include the injection of plasmids, viruses, cell lines, or other vectors containing the respective gene, such as the CSF3 gene. Furthermore, methods for the preparation of transgenic animals have also been described.

For example, the genome can be modified to put the G-CSF gene (CSF3) sequence under the control of a strong promoter that controls expression of the gene. Preferably, the promoter included into the genome of the non-human mammalian animal is an inducible promoter, which allows for the controlled activation of G-CSF gene expression. A suitable inducible promoter may be, e.g. a doxycycline-inducible promoter. Alternatively, the promoter may be a tissue-specific promoter, such as the albumin promoter.

Methods for incorporating a modified promoter sequence are known in the art and include, as described above, the use of a targeting vector that will be introduced into embryonic stem cells of the animal. The knocked-out embryonic stem cells are then inserted into a blastocyst of the animal to generate offspring, which is then interbred to provide homozygous mice. Alternatively, methods for promoter modification in the genome may comprise the CRISPR/Cas9 system.

Apart from modifications of the genome of the animal, it will also be possible to achieve production of a protein, which is effective in mobilizing leukocytes and/or neutrophils in a non-human animal by relying on viral or non-viral expression vectors. Expression vectors that allow for the expression of a transgene in a non-human animal, such as a mouse, are known. Useful mammalian expression vectors have been described in the prior art and such expression systems are purchasable by different manufacturers. Useful expression systems include plasmid- or viral vector based systems, e.g. LENTI-Smart (InvivoGen), GenScript Expression vectors, pAdVAntage (Promega), ViraPower Lentiviral, Adenoviral Expression Systems (Invitrogen) and adeno-associated viral expression systems (Cell Biolabs).

A suitable mammalian expression vector will normally comprise a promoter, which is functionally linked to the nucleic acid encoding the leukocyte and/or neutrophil mobilizing protein. The promoter sequence must be compact and ensure a strong expression. Suitable promoters include, but are not limited to, the cytomegalovirus promoter (CMV), the Spleen Focus Forming Virus (SFFV) U3 promoter and the adenoviral major late promoter (Ad MLP). As an optional component, the mammalian expression vector may include a suitable enhancer element for increasing the expression level. Examples include the SV40 early gene enhancer (Dijkema et al (1985) EMBO J. 4: 761) and the enhancer of the long terminal repeat (LTR) of Rous Sarcoma Virus (Gorman et al. (1982b) Proc. Natl. Acad. Sci. 79: 6777). The expression vector also optionally comprises transcription termination sequences and polyadenylation sequences for improved expression of the gene encoding the leukocyte and/or neutrophil mobilizing protein. Suitable transcription terminator and polyadenylation signals can, for example, be derived from SV40 (Sambrook et al (1989), Molecular Cloning: A Laboratory Manual). Any other element which is known in the art to support efficient expression may be added to the expression vector, such as the Woodchuck hepatitis post-transcriptional regulatory element (wPRE). The vector may be a vector that can be administered to the animal by injection.

In a particularly preferred aspect, the expression vector is a viral expression vector. Viral vectors typically comprise a viral genome in which a portion of the native sequence has been deleted in order to introduce a heterogeneous polynucleotide without destroying the infectivity of the virus. Due to the specific interaction between virus components and host cell receptors, viral vectors are highly suitable for efficient transfer of genes into target cells. Suitable viral vectors for facilitating gene transfer into a mammalian cell or organism are well known in the art and can be derived from different types of viruses, for example, from a retrovirus, adenovirus, adeno-associated virus (AAV), orthomyxovirus, paramyxovirus, papovavirus, picornavirus, lentivirus, herpes simplex virus, vaccinia virus, pox virus or alphavirus. For an overview of the different viral vector systems, see Nienhuis et al., Hematology, Vol. 16: Viruses and Bone Marrow, N.S. Young (ed.), 353-414 (1993).

For example, the vector to be used for expressing the leukocyte and/or neutrophil mobilizing protein is an adeno-associated viral (AVV) vector, such as an AAV vector selected from the group consisting of serotype 1, 2, 3, 4, 5, 6, 7, 8, 9, and 10 or chimeric AAV derived thereof, which will be even better suitable for high efficiency transduction in the tissue of interest (Wu et al., 2006, Mol Therapy 14:316-27; Bowles et al., 2012, Mol Therapy 20:443-455). Upon transfection, AAV elicits only a minor immune reaction (if any) in the host. Moreover, in contrast to other vector systems AAV vectors are also able to efficiently pass from the blood into terminally differentiated cells. Therefore, AAV is highly suited for gene transfer approaches. For transduction in mice, AAV serotype 6 and AAV serotype 9 are particularly suitable. Thus, in a preferred embodiment of the invention, the sequence encoding a protein, which is effective in mobilizing leukocytes and/or neutrophils is part of an AAV serotype 6 vector. In a further preferred embodiment, the sequence encoding a protein, which is effective in mobilizing leukocytes and/or neutrophils is part of an AAV serotype 9 vector.

Recombinant viral vectors can be generated according to standard techniques. For example, recombinant adenoviral or adeno-associated viral vectors can be propagated in human 293 cells (which provide E1A and E1B functions in trans) to titers in the range of 107-1013 viral particles/mL. Prior to their in vivo application viral vectors may be desalted by gel filtration methods, such as sepharose columns, and purified by subsequent filtering. Purification reduces potential deleterious effects in the subject to which the vectors are administered. The administered virus is substantially free of wild-type and replication-competent virus. The purity of the virus can be proven by suitable methods, such as sodium dodecyl sulphate-polyacrylamide gel electrophoresis (SDS-PAGE) followed by silver staining. This is applicable for both AAV and adenoviral vectors.

Transduction of the vectors into the non-human mammalian animal can be achieved by systemic application, e.g., by intravenous (including hydrodynamic tail vein injection), intraarterial or intraperitoneal delivery of a vector. In a preferred embodiment, the vectors are administered systemically.

In a particularly preferred aspect, the G-CSF comprises or consists of the sequence set forth in SEQ ID NO:1. It is also possible to use variants of the growth factor depicted in SEQ ID NO:1 as long as these variants have retained a substantial part of the leukocyte and/or neutrophil mobilizing activity. For example, if a variant of the enzyme set forth in SEQ ID NO:1 is used which includes several amino acid substitutions, care must be taken that these substitutions do not result in a significant loss of growth factor activity. The growth factors to be used must be active, which means that they have retained at least part of the leukocyte and/or neutrophil mobilizing activity of the G-CSF depicted in SEQ ID NO:1. Preferably, the growth factor to be used according to the invention has retained 50%, 60%, 70%, 80%, 90%, 95%, 96%, 97%, 98%, or 99% of the activity of the growth factor set out in SEQ ID NO:1. Methods that allow a comparison of the activity of a variant growth factor with the growth factor of SEQ ID NO:1 are within the routine skills of a skilled person and include measuring the ability of mobilizing leukocytes and/or neutrophils from bone marrow into circulation after administration into an animal under identical conditions.

Active variants of the amino acid sequence of SEQ ID NO:1 may be expressed in the animal which include sequence differences relative to the amino acid sequence depicted in SEQ ID NO:1. For example, a growth factor from another species may be used, preferably from a closely related mammalian species. For example, when using a DNase-deficient mouse, it will be possible to modify the animal to overexpress G-CSF of rat, hamster or rabbit origin. Thus, in one embodiment, the sequence that is overexpressed is a transgene, i.e. the sequence is not naturally present in the mammalian animal. Variants of the amino acid sequence of SEQ ID NO:1 typically differ from the sequence of SEQ ID NO:1 by one or more deletions, substitutions or additions of amino acids within the polypeptide of SEQ ID NO:1. Accordingly, one or more amino acids of the growth factor in SEQ ID NO:1 may be substituted or deleted as long as such modification does not or not significantly impair the growth factor activity of the resulting variant.

Generally, any amino acid residue of the amino acid sequence shown in SEQ ID NO:1 can be replaced by a different amino acid, provided that the resultant variant is still an active growth factor polypeptide with leukocyte and/or neutrophil mobilizing activity. In particular, the growth factor depicted in SEQ ID NO:1 may be modified by the substitution of a total of 1, 2, 3, 4, 5, 6, 7, 8, 9, 10, 15, 20, 25, 30, 35, 40, 45 or 50 amino acids, and in some embodiments even in up 55 amino acids of the growth factor depicted in SEQ ID NO:1. Preferably, these substitutions are not relevant for the growth factor activity of the polypeptide.

It is particularly preferred that substitutions are conservative substitutions, i.e. substitutions of one or more amino acid residues by an amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids, which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

The amino acids, which can be used for replacing the respective amino acids in the naturally occurring murine G-CSF are generally not limited to specific amino acids. In principle, any other proteinogenic or non-proteinogenic amino acid may be used for substituting the naturally occurring amino acid in the respective position of the G-CSF. Preferably, the amino acids found in the original G-CSF can be replaced by any other naturally occurring, proteinogenic amino acid. As used herein, proteinogenic amino acids are those 23 amino acids, which are regularly found in naturally occurring polypeptides. Preferably, the amino acids are L-amino acids. However, also D-amino acids may be useful for replacing the amino acids in the original polypeptide of SEQ ID NO:1.

Alternatively, the amino acids used for replacing the amino acids in the naturally occurring G-CSF may be non-proteinogenic amino acids, i.e. amino acids, which are not found in naturally occurring polypeptides. These non-proteinogenic amino acids include, for example, α-aminoadipic acid, β-aminoadipic acid, α-aminobutyric acid, α-aminoisobutyric acid, β-alanine, 4-aminobutyric acid, 5-aminovaleric acid, 6-aminohexanoic acid, 8-aminooctanoic acid, 9-aminononanoic acid, 10-aminodecanoic acid, 12-aminododecanoic acid, α-aminosuberic acid, β-cyclohexylalanine, citrulline, dehydroalanine, α-cyclohexylglycine, propargylglycine, pyroglutamic acid, 4-benzoylphenylalanine, δ-hydroxylysine, 4-hydroxyproline, allo-isoleucine, lanthionine (Lan), norleucine, norvaline, ornithine, phenylglycin, pipecolic acid, sarcosine, 1,2,3,4-tetrahydro-isochinoline-3-carboxylic acid, allo-threonine, thiazolidine-4-carboxylic acid, γ-aminobutyric acid (GABA), iso-cysteine, diaminopropionic acid, 2,4-diaminobutyric acid, 3,4-diaminobutyric acid, biphenylalanine and 4-fluoro-phenylalanine. Also included by the term "non-proteinogenic amino acids" are derivatives of the above-mentioned proteinogenic amino acids wherein a side-chain has been modified, for example, by a methylene group, thereby providing e.g. homomethionine, homoserine, homoproline, homothreonine, homotryptophane, homotyrosine, homohistidine and homolysine.

Polypeptides which differ from the sequence depicted in SEQ ID NO:1 by the insertion of one or more additional amino acids are also considered variants in the context of the present invention. Such insertions can be made at any position of the polypeptide shown in SEQ ID NO:1. Similarly, variants also include polypeptides in which one or more amino acids have been deleted relative to the polypeptide shown in SEQ ID NO:1. In principle, such deletions can be applied to any amino acid position of the sequence of SEQ ID NO:1.

According to the invention, the variant of the sequence of SEQ ID NO:1 shows a high degree of sequence identity with the sequence of SEQ ID NO:1. The amino acid identity will be at least about 80%, 85%, 90%, 91%, 92%, 93%, 94%, 95%, 96%, 97%, 98% or 99% when compared in an optimal alignment, for example, by the program BESTFIT using standard parameters. For example, a sequence identity of 90% means that 90% of the amino acids of an analyzed amino acid sequence stretch are identical to the sequence of the reference amino acid sequence depicted in SEQ ID NO:1. Methods and computer programs for determining amino acid sequence identity are well known in the art.

Also encompassed by the invention are fragments of the G-CSF shown in SEQ ID NO:1 as well as active fragments of the above-described variants of the G-CSF shown in SEQ ID NO:1, provided that these fragments are active growth factors. Active fragments of the sequence shown in SEQ ID NO:1 or its variants are polypeptides that differ from the amino acid sequence shown in SEQ ID NO:1 or from the respective variant sequence by the absence of one or more amino acids at the N-terminus and/or the C-terminus of the polypeptide. For example, a fragment of the sequence of SEQ ID NO:1 may differ from the sequence of SEQ ID NO:1 by the lack of about 5, 10, 15, 20, 25, 30, 35, 40, 45 or 50 amino acids at the N-terminus and/or the C-terminus, provided that such fragment retains at least a part of the leukocyte and/or neutrophil mobilizing activity of the original full-length growth factor depicted in SEQ ID NO:1. Likewise, a fragment of a variant of SEQ ID NO:1 may differ from said variant sequence by the lack of about 5, 10, 15, 20, 25, 30, 35, 40, 45 or 50 amino acids at the N-terminus and/or the C-terminus, provided that the fragment still has leukocyte and/or neutrophil mobilizing activity.

According to the invention, overexpression of the sequence encoding the protein that is effective in increasing leukocytes and/or neutrophils results in the formation of neutrophils extracellular traps (NETs).

Apart from the overexpression of an exogenous protein, the animal may have been modified to overexpress the endogenous CSF3 gene. In a preferred embodiment, the endogenous CSF3 is overexpressed by genetically inactivating CD18 or LFA-1. Inactivation of one or both of these genes leads to elevated levels of G-CSF and neutrophils in circulation (Stark et al. (2005), Immunity, 22:285-294). Alternatively, the induction of expression of endogenous CSF3 can be caused by the infusion or over-expression of interleukin-17A (Stark et al. (2005), Immunity, 22:285-294). In addition, chronic delivery of G-CSF protein via e.g. an osmotic pump could cause chronically and strongly increased levels of leukocytes and/or neutrophils in circulation.

In a particularly preferred aspect, a non-human mammalian animal is provided which has been genetically modified to
(a) comprise an inactivated gene encoding DNase1,
(b) comprise an inactivated gene encoding DNase1l3, and
(c) overexpress a sequence encoding a protein that is effective in mobilizing leukocytes and/or neutrophils, preferably murine G-CSF.

In another aspect, the invention relates to the use of a non-human mammalian animal as described herein above as a model for studying inflammation and/or a disease associated with inflammation. In particular, the invention relates to the use of a non-human mammalian animal as described above for identifying or validating targets for diagnostic or therapeutic agents. Such agents can be agents that are useful for diagnosing or treating inflammation and/or a disease associated with inflammation. Preferably, inflammation to be studied in the model comprises the formation of neutrophils extracellular traps (NETs). Even more preferably, said NETs are intravascular NETs.

In yet another aspect, the invention relates to the use of a non-human mammalian animal as described herein above as a model for drug candidate testing. In a preferred aspect, drug candidate testing comprises testing whether said drug candidate modifies the formation or degradation of neutrophil extracellular traps (NETs), such as intravascular NETs.

The invention also provides a method for testing an anti-inflammatory drug candidate, said method comprising
(a) providing a non-human mammalian animal as described herein above;
(b) inducing or mimicking inflammation in said non-human mammalian animal by overexpressing a gene encoding a protein that is effective in increasing leukocytes and/or neutrophils, preferably G-CSF, such as murine G-CSF;
(c) administering the anti-inflammatory drug candidate to said non-human mammalian animal; and
(d) evaluating whether said drug candidate is effective for inhibiting, reducing or ameliorating inflammation.

The invention also provides a method for testing an anti-inflammatory drug candidate, said method comprising
(a) providing a non-human mammalian animal as described herein above;
(b) inducing the formation of NETs, such as intravascular NETs;
(c) administering the drug candidate to said non-human mammalian animal; and
(d) evaluating whether said drug candidate modifies the formation or degradation of NETs.

The non-human mammalian animal that can be used in the above methods is described elsewhere herein. The drug candidate used in the methods of the present invention can include all different types of organic or inorganic molecules, including peptides, oligo- or polysaccharides, fatty acids, steroids, and the like. Typically, the drug candidates will be 1small molecules with less than about 2,500 daltons, less than 2000 daltons, less than 1500 daltons, less than 1000 daltons, or less than 500 daltons. Candidate compounds for use in methods can be provided in the form of libraries which comprise a high number of synthetic or natural compounds.

### DESCRIPTION OF THE FIGURES

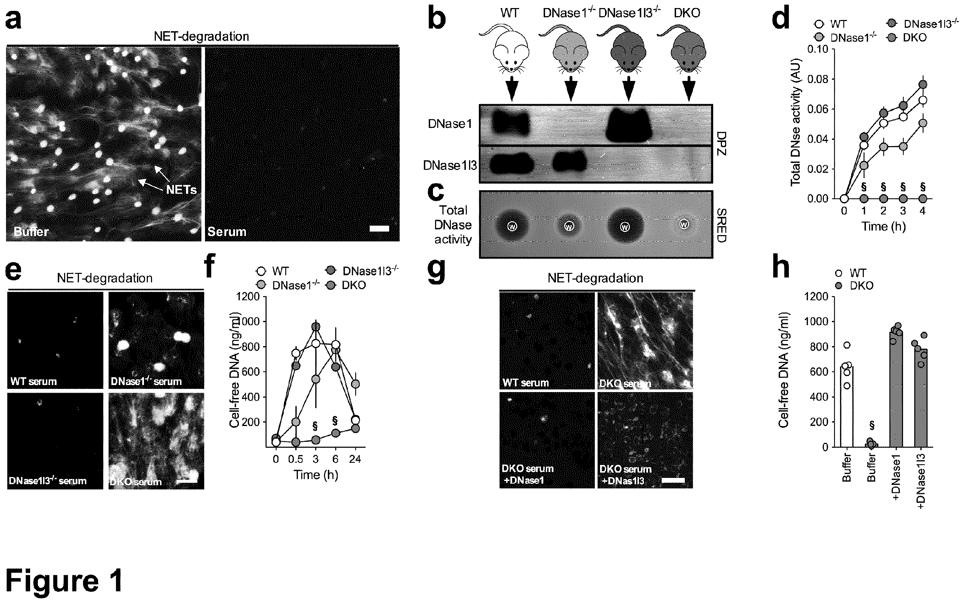

**Figure 1** **shows that DNase1 and DNase1l3 in murine serum degrade NETs.** (**a**) DNA-staining of activated neutrophils shows NETs (arrows) in samples incubated with buffer, whereas incubation with 10 % WT serum for 3 hours degrades NETs. (**b**) DPZ and (**c**) SRED detects the activity of DNase1 and DNase1l3 in WT serum, DNase1l3 in DNase1^{-/-} serum, DNase1 in DNase1l3^{-/-} serum. No DNase activity is detected in DKO serum. (**d**) Quantification of DNase activity shown in panel c over time (n = 5; §: p < 0.001 vs. all other groups). (**e**) NETs are degraded by serum from WT, DNase1^{-/-}, DNasel3^{-/-} mice, but stable in serum from DKO mice (Scale bar: 50 µm). (**f**) Quantification of cell-free DNA fragments in the supernatant of NETs incubated with sera from indicated strains. Sera from DKO mice do not generate cell-free DNA. (n = 4; §: p < 0.001 vs. all other groups). (**g**) Supplementation of DKO serum with recombinant DNase1 or DNase1l3 restores NET-degradation (Scale bar: 50 µm) and (h) the generation of cell-free DNA to WT levels (§: p < 0.001 vs. all other groups).

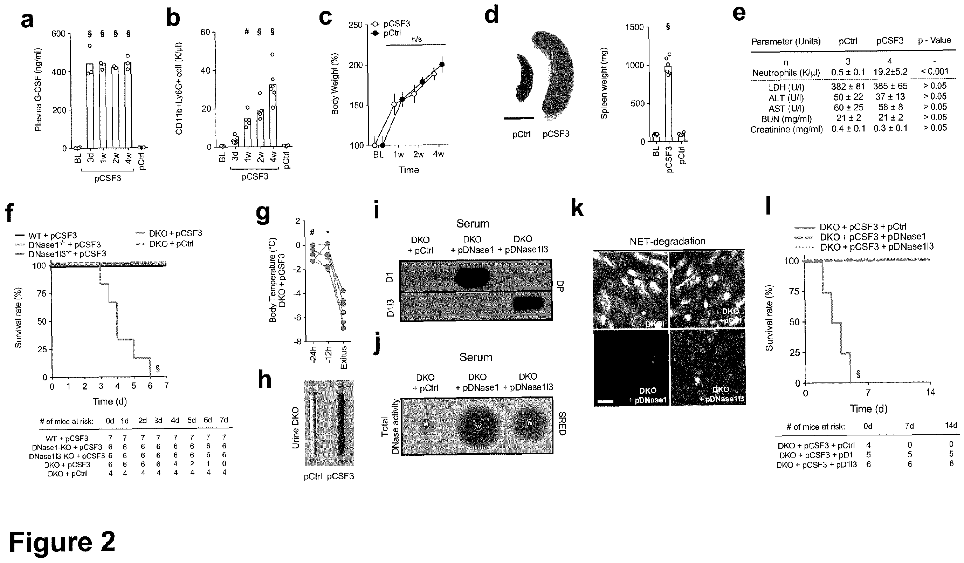

**Figure 2** **shows that extracellular DNases are required for survival of experimental neutrophilia.** (**a**) Four-week old WT mice were injected with a CSF3-expression plasmid (pCSF3) or with a control plasmid (pCtrl). (**a**) G-CSF levels are stably increased 3 days after the injection (§: p < 0.001 vs. base line, BL). (**b**) CD11b/Ly6G-double positive cells in blood steadily increase after G-CSF expression, indicating neutrophilia (#: p < 0.01, §: p < 0.001 vs. BL). (**c**) Mice treated with pCSF3 and pCtrl show a similar growth in body weight (n/s, not significant, pCSF3 vs. pCtrl). (**d**) Mice injected with pCSF3 develop splenomegaly after 4 weeks (scale bar: 1 cm; §: p < 0.001 vs. BL). (**e**) Mice treated with pCSF3 and pCtrl show similar plasma levels of LDH, ALT, AST, BUN, and creatinine. (**f**) Expression of pCSF3 is lethal in DKO mice, but not in WT, DNase1^{-/-}, DNase1l3^{-/-} mice. DKO survive the injection of pCtrl. (§: p < 0.001 vs. all other groups). (**g**) Expression of pCSF3 causes in DKO mice a sudden and rapidly progressing hypothermia (*: p < 0.05, #: p < 0.01 vs. exitus). (**h**) Photograph of glass capillaries filled with urine illustrates hematuria in DKO mice expressing pCSF3. Genetic reconstitution by infection of an expression plasmid containing DNase1 (pDNase1) or DNase1l3 (pDNase1l3) restores the activity of DNase1 and DNase1l3 in serum as detected by (**i**) DPZ, (**j**) SRED, and (**k**) *in vitro* NET-degradation (Scale bar: 50 µm). (**l**) Survival of DKO mice injected with a mixture of pCSF3 and pCtrl, pDNase1, or pDNase1l3. Co-expression of pDNase1 or pDNase1l3 prevents mortality CSF3-induced mortality (§: p < 0.001 vs. all other groups).

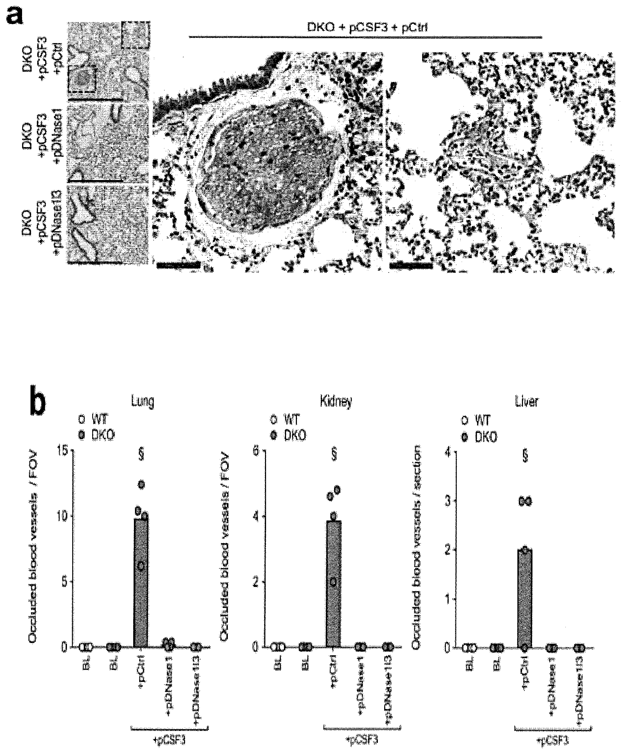

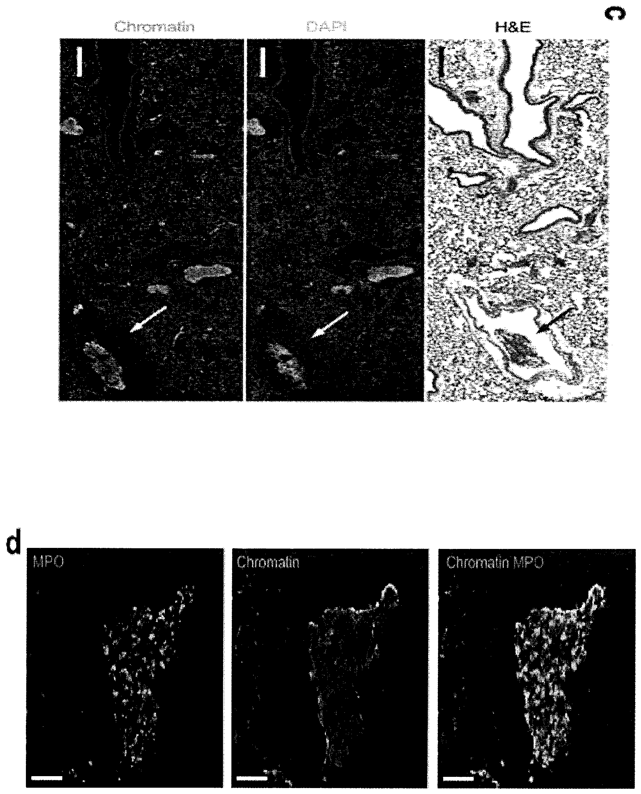

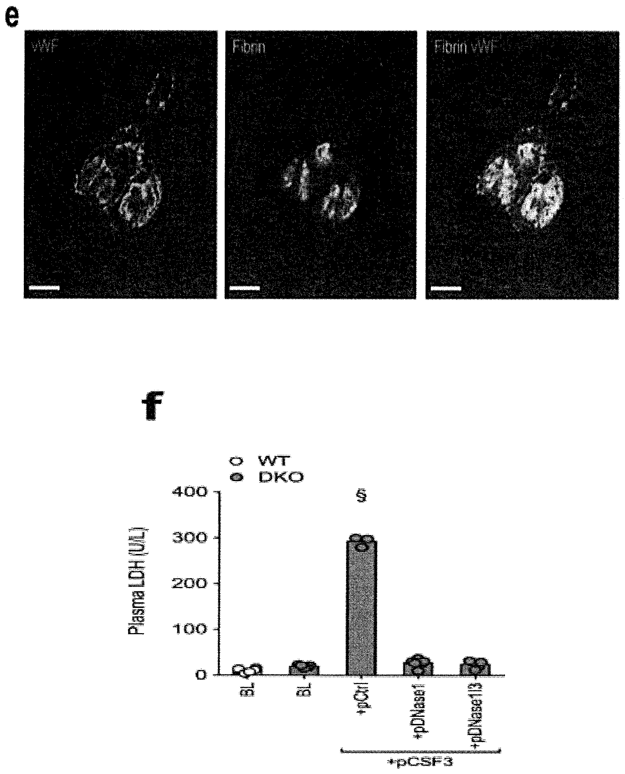

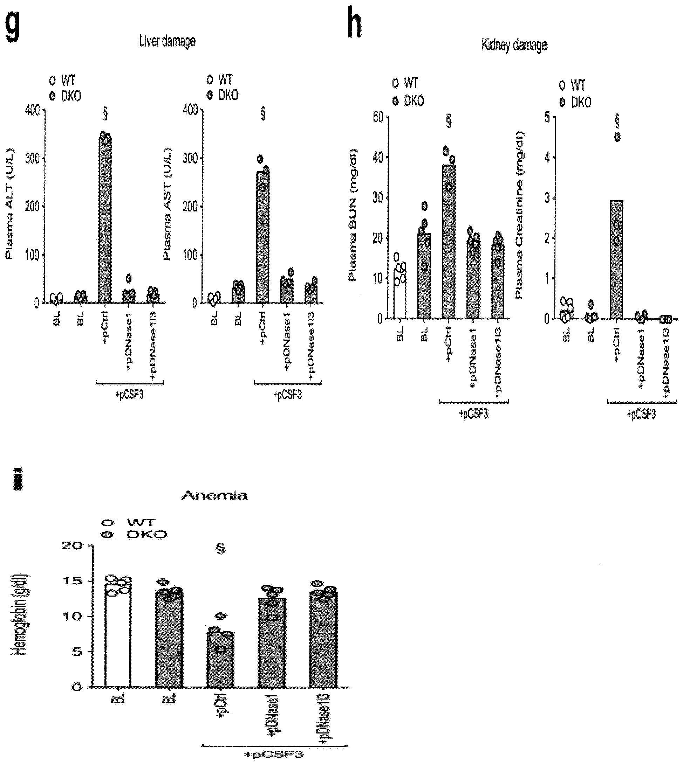

**Figure 3** **shows that extracellular DNases prevent intravascular DNA-clots and multi-organ damage in experimental neutrophila.** (**a**) H&E stainings of lung sections of DKO mice expressing pCSF3 along with pCtrl, pDNase1, or pDNase1l3. Scale bar: 500 µm in overview, 50 µm in magnification. Co-expression of pCSF3 and pDNase1 or pDNase1l3 prevents the formation intravascular hematoxylin-rich clots. (**b**) Quantification hematoxylin-rich clots in lung, kidney, and liver (§: p < 0.001 vs. all other groups). (**c**) Intravascular hematoxylin-rich clots stain for DNA (DAPI) and chromatin (Scale bar: 200 µm). Immunostaining for (**d**) chromatin and myeloperoxidase (MPO) shows abundant NETs as well as (**e**) traces of von Willebrand factor (vWF) and fibrin (Scale bar: 50 µm). Plasma analysis showed elevated levels of (**e**) LDH, (**f**) AST and ALT, (**g**) BUN and creatinine, and (**h**) anemia in DKO mice expressing pCSF3 along with pCtrl, but not in untreated WT and DKO mice (BL) or DKO mice expressing pCSF3 along with pDNase1 or pDNase1l3 (p < 0.001 vs. all other groups).

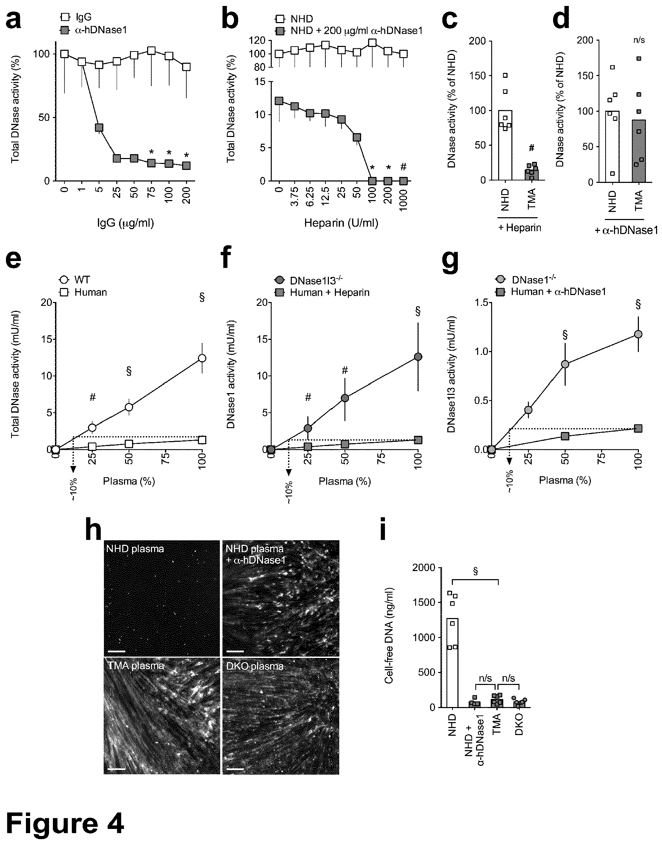

**Figure 4** **shows that mice lacking DNase1 and DNase1l3 and patients with thrombotic microangiopathies degrade NETs inefficiently.** (**a - g**) DNase activity in human and murine plasma quantified by SRED. (**a**) Supplementation of plasma from normal healthy donors (NHD) with polyclonal antibodies against human DNase1 (α-hDNase1), but not with IgG, blocks the DNA degrading activity in a concentration-dependent manner (*: p < 0.05 vs. IgG). (**b**) Plasma supplemented with 200 µg/ml α-hDNase1 and heparin, an inhibitor of DNase1l3, blocks residual DNase activity in plasma supplemented with 200 µg/ml α-hDNase1 (*: p < 0.05 vs. NHD). (**c**) Plasma from patients with acute TMA supplemented with 500 µg/ml heparin shows a reduced activity of DNase1 (#: p < 0.01 vs. NHD). (**d**) Plasma supplemented with 200 µg/ml α-hDNase1 shows a similar DNase1l3 activity in TMA patients and NHD (n/s, not significant vs. NHD). (**e-f**) Comparison of DNase activity in serial dilutions of plasma from mice and NHD. (e) Total DNase activity of WT mice is approximately 10-times higher than in NHD. (**f**) DNase1 activity in plasma from DNase113^{-/-} mice is approximately 10-fold higher than human plasma supplemented with 500 µg/ml heparin. (**g**) DNase1l3 activity in plasma from DNase1^{-/-} mice is approximately 10-fold higher than human plasma supplemented with 200 µg/ml α-hDNase1. (**h-i**) NET-degradation. (**h**) Activated neutrophils incubated with plasma from NHD alone or supplemented with 200 µg/ml α-hDNase1, plasma from TMA patients, and plasma from DKO mice (Scale bars: 200 µm). (**i**) Cell-free DNA in supernatants of activated neutrophils incubated with plasma from indicated sources (§: p < 0.001 vs. NHD). NET-degradation by human plasma is dependent on DNase1. DNase1l3 in human plasma is not sufficient to degrade NETs. Plasma from patients with thrombotic microangiopathies and DKO mice cannot degrade NETs.

### EXAMPLES

The experiments referred to below were approved by the Hamburg State Administration for animal research. All environmental parameters within the animal facility were in compliance with the German Law for the Care and Use of Laboratory animals.

### Example 1: Analysis of DNase activity in DNase-deficient mice.

We used DNase1- and DNase1l3-deficient mice with a C57Bl6-genetic background (Napirei et al. (2000), see above; Mizuta et al. (2013) PloS one, 8:e80223) to generate mice lacking both DNases. To characterize the DNase activity in these animals, we applied the denaturing PAGE zymography (DPZ) method, as previously described with modifications (Napirei, et al. (2009) FEBS J 276 (4): 1059-73). In brief, SDS-PAGE gels were prepared with 4% (v/v) stacking gels without DNA and 10% (v/v) resolving gels containing 200 µg/mL of salmon testes DNA. For DNase1 detection, 0.5 µl of murine serum were mixed with 14.5 µL of water and 5 µl SDS gel-loading buffer (BioRad), boiled for 5 min, and loaded onto the gels. The PageRuler Prestained Protein Ladder (MBI Fermentas, St. Leon-Rot, Germany) was used as molecular marker. Electrophoresis was carried out at 120 V using Tris/glycine electrophoresis buffer (25 mM Tris, 192 mM glycine, 0.1% (w/v) SDS, pH 8.7). After electrophoresis, proteins were refolded by incubating the gels overnight at 37°C in a solution containing 5% (w/v) milk powder, 10 mM Tris/HCl pH 7.8, 3 mM CaCl₂, 3 mM MgCl₂, 100 U/mL penicillin and 100 µg/mL streptomycin. Next, the gels were transferred to a buffer containing 10 mM Tris/HCl pH 7.8, 3 mM CaCl₂, 3 mM MgCl₂, 100 U/mL penicillin, 100 µg/mL streptomycin and 1x SYBR Safe. Gels were incubated for 24 houors at 37°C. The fluorescence was recorded by a fluorescence scanner.

For DNase1l3 detection, 2 µl of serum were mixed with 12 µL of water, 1 µl of betamercaptoethanol (BME) and 5 µl SDS gel-loading buffer, boiled for 5 min, and loaded onto the gels. Electrophoresis was carried out as before. SDS was removed by washing the gels with 10 mM Tris/HCl pH 7.8 for 30 min at 50°C twice, and the proteins were refolded by incubating the gels 48 hours at 37°C in a solution containing 10 mM Tris/HCl pH 7.8, 1 mM BME, 100 U/mL penicillin and 100 µg/mL streptomycin. Next, the gels were incubated for 48 hours at 37°C in a buffer containing 10 mM Tris/HCl pH 7.8, 3 mM CaCl₂, 3 mM MnCl₂, 1 mM BME, 100 U/mL penicillin, 100 µg/mL streptomycin. SYBR Safe was then added to a concentration of 1x and fluorescence was recorded by a fluorescence scanner.

To measure total DNase activity, we dissolved 55 µg/ml DNA from salmon testes (Sigma-Aldrich) in buffer with 20 mM MES pH 6.5, 10 mM MnCl₂, 2 mM CaCl₂, and 2x SYBR Safe (Life Technologies) in distilled water. The DNA solution was heated at 50°C for 10 min and mixed with an equal volume of 2% agarose GP-36 (Nacalai Tesque). The mixture was poured into plastic trays and stored at room temperature until solidification. Two µl of sample (e.g. murine serum) were applied to wells of 1.0 mm radius. Gels were incubated for up to 24 hours at 37°C in a humid chamber, the fluorescence of the gels was recorded with a fluorescence scanner (Molecular Imager FX, Bio-Rad). Image J (NIH) was used for the quantification of the area and intensity of the circles reflecting DNase activity. Absolute units were obtained upon extrapolation against a standard curve obtained by diluting recombinant human DNase1 (Pulmozyme, Roche) in HBSS+ containing 0.1% BSA.

### Results:

Murine and human serum degrades the DNA-backbone of NETs (**Fig 1a**). We therefore speculated that circulating DNases disarm NETs during inflammation. We fractionated murine serum and detected two DNA-degrading enzymes corresponding to DNase1 and DNase1l3 (**Fig. 1b**) (Napirei, et al. (2000) Nat. Genet. 25 (2): 177-81. Sisirak, et al. (2016) Cell 166 (1): 88-101). In mice, DNase1 and DNase1l3 are present in serum at steady-state-conditions, but independently expressed by non-hematopoietic tissues (Napirei, et al. (2004) Biochem. J. 380 (Pt 3): 929-37) or macrophages and dendritic cells (Sisirak, et al. (2016) Cell 166 (1): 88-101), respectively. We generated mice lacking both DNases (DKO) and detected DNA-degrading activity in WT, DNase1^{-/-}, and DNase1l3^{-/-} sera, but not in DKO sera (**Fig. 1c****, d**).

### Example 2: In vitro NET degradation assay

NET-degradation was analyzed according to the protocol of Hakkim et al (Hakkim, et al. (2010) Proceedings of the National Academy of Sciences of the United States of America 107 (21): 9813-8) with modifications. Purified neutrophils in serum-free Dulbecco's Modified Eagle's Medium (DMEM; Life Technologies) were seeded to sterile 96-well plates (Falcon, BD Technologies, Heidelberg, Germany) at a concentration of 5 x 10⁴ cells per well. To induce NET-formation, neutrophils were activated with 100 nM phorbol 12-myristate 13-acetate (PMA; Sigma-Aldrich) for 4 h at 37°C with 5% CO₂ and humidity. We added adding phosphate buffered saline (PBS, naive NETs) or 2% paraformaldehyde (PFA; Sigma Aldrich, fixed NETs) in PBS to the NETs and stored (PFA; Sigma Aldrich, fixed NETs) and stored the 96-well plates overnight at 4 °C. We considered PBS- and PFA-treated NETs as naive NETs and fixed-NETs, respectively. Thereafter, NETs were washed with PBS, treated for 5 minutes with PBS containing 0.5% Triton X-100, and washed with PBS again. NETs were incubated with 10% murine serum or plasma, human citrated plasma, or culture supernatants of HEK cells expressing murine DNase1 or DNase1l3. All samples were diluted 10-fold in HBSS with divalent cations (HBSS+; Life Technologies) and supplemented with 10 µM PPACK (Santa Cruz) in the case of citrated plasma. NETs were allowed to be degraded for different time at 37°C with 5% CO₂ and humidity as indicated in the figures or figure legends. We collected supernatant and stopped NET-degradation by adding 2% PFA in PBS for 1 hours at room temperature. For analysis of NET-degradation, (non-degraded) NETs were then labeled fluorescently by adding 2 µM of the fluorescent DNA dye SytoxGreen (Life Technologies. Images of fluorescently stained nuclei and NETs were acquired with an inverted fluorescence microscope (Zeiss Axiovert 200M, Oberkochen, Germany). In addition, we quantified the fragments of NET-DNA in the supernatants (cell-free DNA), which are generated during NET-degradation. In brief, culture supernatants were diluted with DNAq buffer (0.1% BSA and 2 mM EDTA in PBS) and supplemented with 1 µM SytoxGreen to label DNA. Fluorescence was recorded using a MTP reader (Excitation: 485 nm; Emission: 535 nm). DNA concentrations were calculated based on a standard curve of known concentrations of DNA (lambda DNA, Thermo Scientific). In parallel, auto-fluorescence and the endogenous DNA-concentrations in human and murine samples and culture supernatant, which were not exposed to NETs, were considered as background.

In selected experiments human plasma was suppelemented with rabbit-polycloncal antibodies against recombinant human DNase1 (Pulmozyme, Roche), generated by Exbio (Praha, Czech Republic), and with heparin to block DNase1 and DNase1l3 activity, respectively.

In addition, plasmids containing the cDNA for DNase1 or DNase1l3, described elsewhere (Napirei, et al. (2009) FEBS J 276 (4): 1059-73), were transfected into Human Embryonic Kidney (HEK) cells with Lipofectamine 3000 (Thermo Scientific, Darmstadt, Germany) in serum-free conditions with DMEM media (Gibco) supplemented with 10% KnockOut serum replacement (Gibco). After 72 hours, supernatants were collected, centrifuged at 500 x g for 10 minutes, sterile filtered, and concentrated by ultracentrifugation with 3K columns (Amicon Ultra, Millipore). Supernants were used to study NET-degradation of recombiant murine DNase1 and/or DNase1l3 *in vitro.*

### Results:

Analysis of NET-degradation *in vitro* showed that NETs are stable in DKO-sera, whereas sera from WT, DNase1^{-/-}, and DNase1l3^{-/-} mice degraded NETs (**Fig. 1e**, f). Supplementation of DKO-serum with recombinant murine DNase1 or DNase1l3 restored the NET-degrading activity (**Fig. 1g**, **h**). *In vitro*, DNase1 preferentially cleaves protein-free DNA, while DNase1l3 targets DNA associated with proteins, such as polynucleosomes (Napirei, et al. (2005) Biochem. J. 389 (Pt 2): 355-64). To test whether DNase1 and DNase1l3 degrade NETs by distinct mechanisms, we cross-linked proteins in NETs using fixative. Fixed NETs were efficiently degraded by DNase1l3^{-/-} sera, but were resistant to degradation by DNase1^{-/-} sera (**not shown**). In line with these results, exposure of NETs to recombinant DNase1 or DNase1l3 showed that both DNases degrade naive NETs (**not shown**), whereas fixed NETs are resistant to DNase1l3 activity (**not shown**). Collectively, these *in vitro* data identify extracellular DNase1 and DNase1l3 in murine serum as redundant NET-degrading enzymes.

### Example 3: In vivo NET degradation

For *in vivo* expression, we used the pLIVE plasmid (Mirus Bio), a vector, which allows a strong and maintained hepatocyte-specific expression of the protein of interest upon delivery. PCR of DNase1 cDNA (Genbank Accession Number NM010061) was performed using the pair of primers DNase1-F 5'- GTCGACATGCGGTACACAGG and DNase1-R 5'-CTCGAGTCAGATTTTTCTGAGTGTCA containing SalI and XhoI restriction sites. PCR of DNase1l3 cDNA (Genbank Accession Number AF047355) was performed using the pair of primers DNase1l3-F 5'-GAAGTCCCAGGAATTCAAAGATGT and DNase1l3-R 5'-GCGTGATACCCGGGAGCGATTG containing BamHI and SacI restriction sites. Both cDNAs were cloned using the T4 ligase (New England Biolabs, Frankfurt, Germany) into the MCS site of the vector pLIVE previously digested with the appropriate enzymes. The DNase1l3 pLIVE vector was subjected to site-directed mutagenesis with the pair of primers mutD1l3-F 5'-AGTCGACTCCCGGCCACCATGTCCCTGCA and its complementary mutD1l3-R 5'- TGCAGGGACATGGTGGCCGGGAGTCGACT in order to match the consensus Kozak sequence. The synthesis of the pLIVE-CSF3 plasmid (pCSF3) was outsourced to Eurofins Genomics. The cDNA of the CSF3 gene (Genbank Accession Number BC120761) was inserted in the MCS between restriction sites SalI and XhoI. Sequence of all the generated vectors was confirmed by double stranded sequencing. As a control we used the parental pLIVE plasmid (pCtrl). Plasmids were amplified and purified from potential contaminations with endotoxin.

The pLIVE-expression vectors described above were administered to mice via the method of hydrodynamic tail vein injection, as described elsewhere. In brief, 50 µg of plasmid were diluted in 0.9% saline in a volume equivalent to 10% of the body mass of the mouse. Mice were anaesthetized with isoflurane (2.5% of Isoflurane, Abbvie) and the plasmid solution was then injected intravenously over 5 to 8 seconds via the tail vein. In rare cases, mice did not fully recover from the injection within the first 24 hours and were excluded from the study.

Mice were injected with 50 µg of pCSF3 or CSF3 to induce G-CSF expression and neutrophilia. For co-expression studies, 50 µg pCSF3 was mixed with 50 µg pCtrl or 50 µg pDNase1 or 50 µg pDNase1l3. A solution containing both plasmids was administered via hydrodynamic tail vein injection. Weight and temperature were monitored every 8 hours during the first week after injection, and daily afterwards. Temperature was measured in the perianal area by a contactless infrared-thermometer (Etekcity). Severe hypothermia was defined as decrease in body temperature of ≥4°C, compared to the body temperature before the plasmid injection. In all cases of hypothermia was accompanied with symptoms of distress. Therefore hypothermic mice were sacrificed and blood, urine, and organs were collected. Non-hypothermic mice did not show any signs of distress and were euthanized at the end of the experiments to collect blood, urine, and organs.

### Results:

To test the requirement of DNase1 or DNase1l3 for NET-degradation *in vivo,* we overexpressed murine *CSF3* in WT, DNase1^{-/-}, DNase1l3^{-/-}, and DKO mice. *CSF3* encodes the granulocyte-colony stimulating factor (G-CSF), which mobilizes neutrophils from the bone marrow to circulation (Semerad, et al. (2002) Immunity 17 (4): 413-23) and stimulates a subpopulation of neutrophils to release NETs *ex vivo* and *in vivo* (Demers, et al. (2012) Proceedings of the National Academy of Sciences of the United States of America 109 (32): 13076-81). We induced G-CSF expression by injecting a liver-specific expression plasmid containing the *CSF3* coding sequence (pCSF3) into 4-week old WT mice. High levels of G-CSF were detectable in plasma 3 days after pCSF3 administration (**Fig. 2a**) and the concentration of circulating neutrophils increased steadily henceforward (**Fig. 2b**). After 2 weeks, we detected approximately 40-fold higher neutrophil counts in G-CSF-overexpressing mice, when compared to animals injected with the control plasmid lacking *CSF3* (pCtrl, **Fig. 2b**). Neutrophilic mice developed increased numbers of resident neutrophils in vital organs (not shown), splenomegaly (**Fig. 2c**), but grew normally (**Fig. 2d**), did not show signs of organ injury (**Fig. 2e**), and were macroscopically indistinguishable from untreated animals (not shown).

We then compared the course of G-CSF-induced neutrophilia in WT mice to DNase1^{-/-}, DNase1l3^{-/-}, and DKO mice. Single KO mice were macroscopically indistinguishable from neutrophilic WT mice (**Fig. 2f**) and untreated controls (not shown). All DKO mice died within 7 days after induction of G-CSF overexpression and developed a rapidly progressing and severe hypothermia with concomitant hematuria (**Fig. 2f****, g**). No such phenotype was observed in DKO mice injected with the control plasmid (**Fig. 2f**). In order to conclude that the lack of both circulating DNases is responsible for this phenotype, we performed several control experiments. An off-target mutation has been recently reported for the DNase1^{-/-} mice used in this study(Rossaint, et al. (2014) Blood 123 (16): 2573-84). We therefore reproduced the phenotype in single and DKO mice derived from an independently generated DNase1^{-/-} strain (Bradley, et al. (2012) Mammalian genome 23 (9-10): 580-6.) (**not shown**). DNase1-and DNase1l3-deficient mice spontaneously develop autoimmunity with features of lupus nephritis^{10,11}. Furthermore, DNase1 and/or DNase1l3 degrade nuclear material generated during cell death (Sisirak, et al. (2016) Cell 166 (1): 88-101), and DNase1l3 has been implicated in B-cell development (Shiokawa, et al. (2007) Cell Death Differ. 14 (5): 992-1000). To rule out actions of DNase1 and DNase1l3 other than degrading extracellular DNA, we treated DKO mice with a liver-specific expression plasmid containing the cDNA of DNase1 (pDNase1) or DNase1l3 (pDNase1l3). Both enzymes contain a secretion sequence (Napirei, et al. (2009) FEBS J 276 (4): 1059-73), and consequently, hepatic expression of DNase1 or DNase1l3 restored their enzymatic activity in circulation (**Fig. 2h****, i**) as well as the NET-degrading activity of serum (**Fig. 2j**). Importantly, DKO mice genetically reconstituted with DNase1 or DNase1l3 in circulation survived G-CSF-induced neutrophilia, did not develop severe hypothermia or hematuria (**Fig. 2k**), and were macroscopically indistinguishable from untreated DKO mice (not shown).

### Example 4: Analysis of pathology

LDH, AST, ALT, Bilirubin, Creatinine and BUN in plasma were analyzed by using standardized kits (Biotron Diagnostics) following the manufacturer instructions. Mouse G-CSF was quantified with a Quantikine ELISA Kit (R&D), following manufacturer's indications. Hemoglobin in EDTA-blood was quantified by an automated hemocytometer (Idexx ProCyte Dx Hematology Analyzer)..To quantify neutrophils, whole blood was incubated on ice for 15 minutes with 0.2 µg of PE-labelled anti-mouse CD11b (M1/70, Biolegend) and 0.5 µg of Alexa Fluor 488 anti-mouse Ly6G (1AB, Biolegend). Sample was then diluted with 0.5 ml PBS and analyzed with the BD FACS Calibur. Data collection and sorting was performed using the CellQuest™ Software (BD immunocytometry systems, CA, USA).

For immunohistochemistry, paraffin-embedded sections were de-paraffinized, rehydrated, and subjected to antigen retrieval for 25 minutes at 100°C in citrate buffer (10 mM sodium citrate, 0.1% Tween, pH 6). Thereafter, sections were blocked for 30 minutes with 2.5% normal goat serum (Vector Labs) followed by incubation with a mouse-on-mouse blocking kit (Vector) for one hour. The sections were then incubated over night at 4°C with 2 µg/ml of the primary antibody against CRAMP (PA-CRLP-100, Innovagen), citrullinated histone 3 (ab5103, Abcam), fibrin-specific (clone 59D8), the complex of histone H2A, H2B, and DNA to detect chromatin, respectively. Sections were incubated with anti-rabbit and anti-mouse conjugated with AlexaFluor488 or AlexaFluor555 (Life Technologies) for 1 hour. After washing, DAPI staining (1 µg/ml) was applied for 2 minutes and washed. Autofluorescence was quenched by 25 minutes incubation with Sudan Black (0.1% in 70% EtOH), and sections were mounted with Fluoromount G (Southern Biotech). Images of fluorescently labeled sections were acquired with an inverted fluorescence microscope (Zeiss Axiovert 200M, Oberkochen, Germany) or a confocal microscope (Leica TCS SP5).

### Results:

Histological analysis of vital organs from hypothermic DKO mice revealed intravascular hematoxylin-rich clots, which fully or partially occluded blood vessels in lungs, liver, and kidneys (**Fig. 3a****, b**). Untreated DKO mice, neutrophilic WT mice, and neutrophilic DKO mice expressing pDNase1 or pDNase1l3 did not show occluded blood vessels (**Fig. 3b**). We observed two distinct staining patterns in intravascular hematoxylin-rich clots: a dotted pattern illustrating nuclei of leukocytes and an abundant diffuse staining pattern covering the space between nuclei (**Fig. 3a**). Positive staining with intercalating DNA dyes and antibodies against DNA-histone-complexes (**Fig. 3c**) identified extracellular chromatin as a major clot component. Extracellular chromatin was mainly derived from NETs, as evidenced by co-localization with markers of neutrophil granules such myeloperoxidase (**Fig. 3d**), cathelin-related antimicrobial peptide (CRAMP, not shown), and by the NET-surrogate marker citrullinated histones (**not shown**). Additionally, the DNA-clots contained von Willebrand factor (vWF) and fibrin (not shown), supporting the previously described pro-thrombotic activity of NETs (Engelmann, et al. (2013) Nat. Rev. Immunol. 13 (1): 34-45). The formation of DNA-clots was associated with organ damage, as evidenced by increased levels of plasma LDH (**Fig 3f**). Elevated plasma levels of ALT and AST, indicated liver damage (**Fig 3g**), while high levels of BUN and creatinine in plasma, and hematuria indicated renal failure (**Fig**. **3h**). In addition, DKO mice developed anemia (**Fig. 3i**). In summary, these data suggest that mice lacking DNase1 and DNase1l3 died of multi-organ damage induced by systemic intravascular DNA-clots comprising NETs.

### Example 5: Comparison of DNase activity in healthy donors, patients, and mice

Plasma from normal healthy donors (NHD) and patients with thrombotic miroangiopathies (Jimenez-Alcazar, et al. (2015) J. Thromb. Haemost. 13 (5): 732-42), as well as mice was analyzed by SRED and in-vitro NET-degradation as outlined in Example 1.

### Results:

The importance of circulating DNases to maintain homeostasis during inflammation is supported by our previous clinical observations. We identified elevated markers of neutrophil activation (Fuchs, et al. (2012) Blood 120 (6): 1157-64) along with a deficiency in circulating DNase1 in plasma from patients with acute thrombotic microangiopathies (TMA), a rare heterogeneous disease associated with infection and autoimmunity (Jimenez-Alcazar, et al. (2015) J. Thromb. Haemost. 13 (5): 732-42). DKO mice subjected to experimental neutrophilia and endotoxemia develop several features of patients with acute TMA, including strongly elevated levels of LDH, thrombocytopenia, anemia, hematuria, and organ damage (Kremer Hovinga, et al. (2010) Blood 115 (8): 1500-11; quiz 662). We therefore questioned whether TMA patients have a dual-deficiency in circulating DNase1 and in circulating DNase1l3. To discriminate DNase1 and DNase1l3 activity, we generated antibodies against human DNase1 (α-hDNase1), which block the enzymatic activity in a concentration-dependent manner in plasma from normal healthy donors (NHD) (**Fig. 4a**). Heparin is a known inhibitor of DNase1l3 (Napirei, et al. (2009) FEBS J 276 (4): 1059-73) and was used to block the DNA-degrading activity by DNase1l3 (**Fig. 4b**). Analysis of heparinized plasma confirmed the deficiency in DNase1 activity in TMA patients compared to healthy individuals (**Fig. 4c**). However, plasma spiked with α-hDNase1 showed a similar DNA-degrading activity in between patients and healthy donors (**Fig. 4d**), indicating normal levels of DNase1l3 activity in acute TMA and suggesting that DNase1 and DNase1l3 are independently regulated in diseased humans.

These data furthermore illustrate a discrepancy between humans and mice. Whereas TMA patients show normal levels of DNase1l3 activity, mice with normal levels of DNase1l3 do not develop vascular occlusions and organ damage in our experimental models. We therefore compared circulating DNase1 and DNase1l3 in mice to humans. Murine plasma showed approximately 10-fold higher DNA-degrading activity than human plasma (**Fig. 4e**). Comparison of plasma from DNase1^{-/-} and DNase1l3^{-/-} mice to plasma from NHD supplemented with α-hDNase1 and heparin, respectively, showed that activity of both DNases is approximately 10-fold higher in mice than in healthy donors (**Fig. 4f****, g**). Finally, we speculated the DNase1l3 activity observed in NHD and TMA patients is not sufficient to degrade NETs efficiently. Indeed, NETs were stable in plasma from NHD supplemented with α-hDNase1 and in plasma from TMA patients. (**Fig. 4h****, i**). Consequently, TMA patients and mice lacking DNase1 and DNase1l3, both cannot degrade NETs efficiently.

## Claims

1. Non-human mammalian animal which has been modified to have in the blood, plasma and/or serum
(a) an increased number of leukocytes and/or neutrophils, and
(b) a reduced activity of the DNase 1 and/or DNase 1-like 3 enzyme.

2. Non-human mammalian animal according to claim 1, wherein said non-human mammalian has been modified to express a sequence encoding a protein that is effective in increasing leukocytes and/or neutrophils in the blood, plasma and/or serum.

3. Non-human mammalian animal according to any of claims 1-2, wherein said protein is the granulocyte-stimulating factor (G-CSF).

4. Non-human mammalian animal according to any of claims 1-3, wherein said mammalian animal is a rodent.

5. Non-human mammalian animal according to claim 4, wherein said mammalian animal is a mouse.

6. Non-human mammalian animal according to any of claims 1-5, wherein said mammalian animal is a mouse and said G-CSF is murine G-CSF.

7. Non-human mammalian animal according to claim 6, wherein said murine G-CSF comprises or consists of the sequence set forth in SEQ ID NO:1, a sequence having at least 80% sequence identity thereto, or an active fragment of any of these.

8. Non-human mammalian animal according to any of claims 2-7, wherein overexpression of the sequence encoding a protein that is effective in increasing leukocytes and/or neutrophils is controlled by an inducible promoter.

9. Non-human mammalian animal according to any of claims 1-8, wherein overexpression of the gene encoding a protein that is effective in increasing leukocytes and/or neutrophils results in the formation of neutrophils extracellular traps (NETs).

10. Non-human mammalian animal according to any of claims 1-9, wherein said non-human mammalian animal has been genetically modified to
(a) have an inactivated gene encoding DNase1,
(b) have an inactivated gene encoding DNase1l3, and
(c) overexpress a sequence encoding a protein that is effective in increasing leukocytes and/or neutrophils, preferably murine G-CSF.

11. Use of a non-human mammalian animal according to any of claims 1-10 for identifying or validating targets for diagnostic or therapeutic agents, preferably agents which are useful for diagnosing or treating inflammation and/or a disease associated with inflammation.

12. Use according to claim 11, wherein said inflammation comprises the formation of neutrophils extracellular traps (NETs), such as intravascular NETs.

13. Use of a non-human mammalian animal according to any of claims 1-10 for drug candidate testing.

14. Use according to claim 13, wherein drug candidate testing comprises testing whether said drug candidate modifies the formation or degradation of neutrophil extracellular traps (NETs), such as intravascular NETs.

15. Method for testing an anti-inflammatory drug candidate, said method comprising
(a) providing a non-human mammalian animal according to any of claims 1-10;
(b) inducing or mimicking inflammation in said non-human mammalian animal by increasing the numbers of leukocytes and/or neutrophils in the blood, plasma and/or serum and reducing the activity of the DNase 1 and DNase 1-like 3;
(c) administering the drug candidate to said non-human mammalian animal; and
(d) evaluating whether said drug candidate is effective for inhibiting, reducing or ameliorating said inflammation.

16. Method for testing an anti-inflammatory drug candidate, said method comprising
(a) providing a non-human mammalian animal according to any of claims 1-10;
(b) inducing the formation of NETs, such as intravascular NETs;
(c) administering the drug candidate to said non-human mammalian animal; and
(d) evaluating whether said drug candidate modifies the formation or degradation of NETs.


SEQUENCE LISTING

<110>  Universitätsklinikum Hamburg-Eppendorf

<120>  ANIMAL MODEL FOR DRUG DEVELOPMENT

<130>  P 101228

<160>  5

<170>  PatentIn version 3.5

<210>  1
<211>  208
<212>  PRT
<213>  Mus musculus

<400>  1

Met Ala Gln Leu Ser Ala Gln Arg Arg Met Lys Leu Met Ala Leu Gln
1               5                   10                  15

Leu Leu Leu Trp Gln Ser Ala Leu Trp Ser Gly Arg Glu Ala Val Pro
            20                  25                  30

Leu Val Thr Val Ser Ala Leu Pro Pro Ser Leu Pro Leu Pro Arg Ser
        35                  40                  45

Phe Leu Leu Lys Ser Leu Glu Gln Val Arg Lys Ile Gln Ala Ser Gly
    50                  55                  60

Ser Val Leu Leu Glu Gln Leu Cys Ala Thr Tyr Lys Leu Cys His Pro
65                  70                  75                  80

Glu Glu Leu Val Leu Leu Gly His Ser Leu Gly Ile Pro Lys Ala Ser
                85                  90                  95

Leu Ser Gly Cys Ser Ser Gln Ala Leu Gln Gln Thr Gln Cys Leu Ser
            100                 105                 110

Gln Leu His Ser Gly Leu Cys Leu Tyr Gln Gly Leu Leu Gln Ala Leu
        115                 120                 125

Ser Gly Ile Ser Pro Ala Leu Ala Pro Thr Leu Asp Leu Leu Gln Leu
    130                 135                 140

Asp Val Ala Asn Phe Ala Thr Thr Ile Trp Gln Gln Met Glu Asn Leu
145                 150                 155                 160

Gly Val Ala Pro Thr Val Gln Pro Thr Gln Ser Ala Met Pro Ala Phe
                165                 170                 175

Thr Ser Ala Phe Gln Arg Arg Ala Gly Gly Val Leu Ala Ile Ser Tyr
            180                 185                 190

Leu Gln Gly Phe Leu Glu Thr Ala Arg Leu Ala Leu His His Leu Ala
        195                 200                 205

<210>  2
<211>  20
<212>  DNA
<213>  Artificial Sequence

<220>
<223>  oligonucleotide primer

<400>  2
gtcgacatgc ggtacacagg                                                   20

<210>  3
<211>  26
<212>  DNA
<213>  Artificial Sequence

<220>
<223>  Oligonucleotide primer

<400>  3
   ctcgagtcag atttttctga gtgtca                                             26

<210>  4
<211>  24
<212>  DNA
<213>  Artificial Sequence

<220>
<223>  Oligonucleotide primer

<400>  4
gaagtcccag gaattcaaag atgt                                              24

<210>  5
<211>  29
<212>  DNA
<213>  Artificial Sequence

<220>
<223>  Oligonucleotide primer

<400>  5
tgcagggaca tggtggccgg gagtcgact                                         29